# EUROPEAN PATENT SPECIFICATION

(11) **EP 4 112 617 B1**
(45) Date of publication and mention of the grant of the patent: **25.11.2026**
(21) Application number: 21761001.3
(22) Date of filing: 26.02.2021
(51) Int. Cl.: C07D 417/14, A61K 31/5377

(54) **METHOD FOR PREPARATION OF HETEROCYCLICAMINE DERIVATIVES**
VERFAHREN ZUR HERSTELLUNG VON HETEROCYCLICAMIN-DERIVATEN
PROCÉDÉ DE PRÉPARATION DE DÉRIVÉS D'AMINE HÉTÉROCYCLIQUE

(30) Priority: 26.02.2020 KR 20200023899; 25.02.2021 KR 20210025655
(43) Date of publication of application: 04.01.2023
(73) Proprietor: Daewoong Pharmaceutical Co., Ltd., Hwaseong-si, Gyeonggi-do 18623 (KR)
(72) Inventor: KIM, Wol Young, Seongnam-si, Gyeonggi-do 13532 (KR); LI, Qing Ri, Suwon-si, Gyeonggi-do 16459 (KR); EOM, Deok Ki, Yongin-si, Gyeonggi-do 17000 (KR); HYUN, Hyae Jung, Seongnam-si, Gyeonggi-do 13568 (KR); PARK, Joon Seok, Yongin-si, Gyeonggi-do 17000 (KR)
(74) Representative: Germain Maureau
(86) International application number: PCT/KR2021/002440
(87) International publication number: WO 2021/172922

(56) References cited:
- EP-A1- 3 845 530
- WO-A1-2012/035055
- WO-A1-2012/035055
- CN-A- 104 628 657
- KR-A- 20190 040 773
- KR-A- 20190 040 773
- KR-A- 20200 024 111
- US-A1- 2017 233 411
- HARLING JOHN D., DEAKIN ANGELA M., CAMPOS SÉBASTIEN, GRIMLEY RACHEL, CHAUDRY LAIQ, NYE CATHERINE, POLYAKOVA OXANA, BESSANT CHRISTI: "Discovery of Novel Irreversible Inhibitors of Interleukin (IL)-2-inducible Tyrosine Kinase (Itk) by Targeting Cysteine 442 in the ATP Pocket", JOURNAL OF BIOLOGICAL CHEMISTRY, AMERICAN SOCIETY FOR BIOCHEMISTRY AND MOLECULAR BIOLOGY, US, vol. 288, no. 39, 9 August 2013 (2013-08-09), US, pages 28195 - 28206, XP055841724, ISSN: 0021-9258, DOI: 10.1074/jbc.M113.474114

## Description

### [TECHNICAL FIELD]

### CROSS-REFERENCE TO RELATED APPLICATION(S)

This application claims the benefit of Korean Patent Application No. 10-2020-0023899 filed on February 26, 2020 and Korean Patent Application No. 10-2021-0025655 filed on February 25, 2021 in the Korean Intellectual Property Office.

The present disclosure relates to a method for preparing a heterocyclic amine derivative.

### [BACKGROUND ART]

ITK(Interleukin-2 Tyrosine Kinase) and BTK(Bruton's Tyrosine Kinase) are a type of tyrosine kinase, together with Tec(tyrosine kinase expressed in hepatocellular carcinoma), RLK(Resting Lymphocyte Kinase) and BMX(Bone-Marrow tyrosine kinase gene on chromosome X), which does not have a TEC-family receptor and acts on various immune responses.

ITK is expressed not only in T cells but also in NK cells and mast cells, and plays an important role in T-cell proliferation and in the production of important cytokines such as IL-2, IL-4, IL-5, IL-10, IL-13 and IL-17 (Schaeffer et al. Nat. Immune 2001, 2, 1183; Fowell et al. Immunity, 1999, 11, 399). T cells are activated by TCR signaling, and the activated T cells produce inflammatory cytokines, and activate B cells and macrophages, causing autoimmune diseases such as RA (Sahu N. et al. Curr Top Med Chem. 2009, 9, 690). Previously, it has been known that Th1 cells are activated by T cells to induce RA diseases, but recently, it has been reported that not only Th17/Treg but also Th1 cells act as a pathogenesis of RA (J Leipe J. et al. Arthritis Rheum. 2010, 62, 2876). In addition, the ITK has been previously developed as an immunotherapeutic drug target such as asthma, but no ITK has been developed as a therapeutic drug for RA (Lo H. Y Expert Opin Ther Pat. 2010, 20, 459). Recently, however, it has been reported to regulate the development of Th17 and Treg cells via ITK-/- mice, and it has ample potential as a therapeutic target for RA (Gomez-Rodriguez J. et al. J. Exp. Med. 2014, 211, 529).

In a study using the ITK inhibitor PRN694, a study on the reduction of TNF-α which is a typical inflammatory cytokine of RA diseases, has been reported, confirming the possibility of development as a therapeutic agent for RA by regulating Th17 expression through ITK inhibition (Zhong Y. et al. THE JOURNAL OF BIOLOGICAL CHEMISTRY 2015, 290, 5960).

BTK acts as a regulator of early B-cell development as well as of mature B-cell activation, signaling and survival. The B-cell is signaled by a B cell receptor (BCR) that recognizes an antigen attached to the surface of an antigen-resenting cell and is activated into a mature antibody-producing cell. However, aberrant signaling via BCR leads to abnormal B-cell proliferation and the formation of pathologic autoantibodies, and thereby can induce cancer, autoimmune and/or inflammatory diseases. Thus, in the abnormal B-cell proliferation, signaling via BCR may be blocked when BTK is deficient. Consequently, inhibition of BTK can block B-cell mediated disease processes, and thus, the use of BTK inhibitors may be a useful approach for the treatment of B-cell mediated diseases.

Furthermore, BTK can be expressed by other cells that may be associated with disease besides B-cells. In one example, BTK is important components for Fc-gamma signaling in bone marrow cells, and is expressed by mast cells. Specifically, BTK-deficient bone marrow-induced mast cells exhibit impaired antigen-induced degranulation, and inhibition of BTK activity is known to be useful for treating pathological mast cell responses such as allergy and asthma (Iwaki et al. J. Biol Chem. 2005280: 40261). In addition, it is known that monocytes from XLA patients, in which BTK activity is absent, decreases in TNF alpha production following stimulation and thus TNF alpha-mediated inflammation could be inhibited by BTK inhibitors (see, Horwood et al., J. Exp. Med. 197: 1603, 2003).

At present, there has been no case where it has been developed as a substance that dually inhibits BTK and ITK. As the BTK inhibitor, however, WO 2008/039218 discloses 4-aminopyrazolo[3,4-d]pyrimidinylpiperidine derivatives, and WO2015/061247 discloses hetero compounds such as pyridine, pyrimidine, pyrazine and pyridazine, and WO2014/055934 discloses pyrimidinyl phenyl acrylamide derivatives. As the ITK inhibitor, WO2005/066335 discloses aminobenzimidazoles, WO2005/056785 discloses pyridones, WO2002/050071 discloses aminothiazole derivatives, and recently, WO2014/036016 discloses benzimidazole derivatives.

WO 2012/035055 A1 discloses compounds of formula (I): 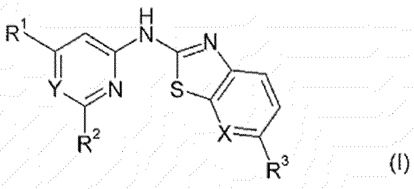 wherein R¹ is hydrogen, -CH₂OR⁴, -CH₂NR⁵R⁶, -CH₂-phenyl, R² is -(NH)₂COCHCH₂, -NR⁷R⁸, -OR⁹ or phenyl and R³ is hydrogen, halo, C₁₋₆alkyl or C₁₋₆alkoxy. These compounds are inhibitors of kinase activity, in particular Itk activity.

Given these circumstances, the present inventors have found that a heterocyclic amine derivative having a chemical structure different from BTK, ITK inhibitors reported so far exhibits excellent BTK and ITK dual-activity inhibitory effect. Thus, the inventors have conducted intensive research on a preparation method capable of preparing a novel heterocyclic amine derivative, and as a result, found that when the preparation method described hereinafter is used, commercial mass production is possible, the yield is improved as a whole, and impurities are reduced, thereby completing the present disclosure.

### [DETAILED DESCRIPTION OF THE INVENTION]

### [Technical Problem]

It is one object of the present disclosure to provide a method for preparing a heterocyclic amine derivative.

### [Technical Solution]

In order to achieve the above objects, the present disclosure provides a preparation method as shown in the following Reaction Scheme 1, and more specifically, provides a preparation method comprising the steps of:
1) preparing a compound represented by the following Chemical Formula 1-3 by reacting a compound represented by the following Chemical Formula 1-1 with a compound represented by the following Chemical Formula 1-2 in the presence of a palladium catalyst and a base;
2) preparing a compound represented by the following Chemical Formula 1-4 by reacting a compound represented by the following Chemical Formula 1-3 in the presence of an acid; and
3) preparing a compound represented by the following Chemical Formula 1 by reacting a compound represented by the following Chemical Formula 1-4 with a compound represented by the following Chemical Formula 1-5 in the presence of a base, wherein the reaction is carried out at a temperature of -10°C or more and less than 0°C:

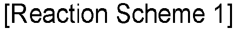

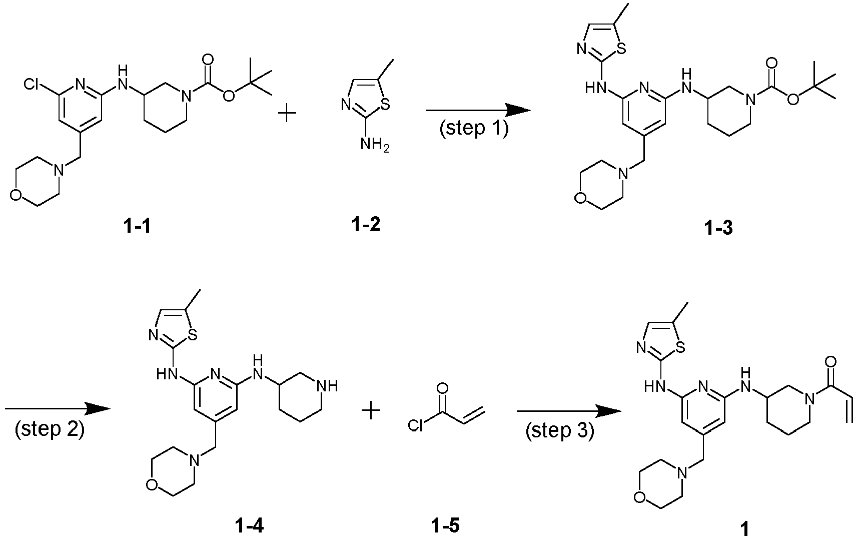

At this time, the compound represented by Chemical Formula 1 may be understood as a concept of all-encompassing the following three types of compounds according to chirality.
(1) (S)-1-(3-((6-((5-methylthiazol-2-yl)amino)-4-(morpholinomethyl)pyridin-2-yl)amino)piperidin-1-yl)prop-2-en-1-one 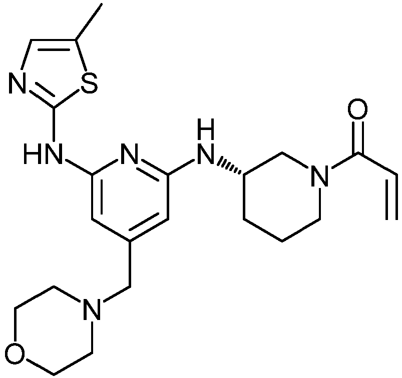
(2) (R)-1-(3-((6-((5-methylthiazol-2-yl)amino)-4-(morpholinomethyl)pyridin-2-yl)amino)piperidin-1-yl)prop-2-en-1-one 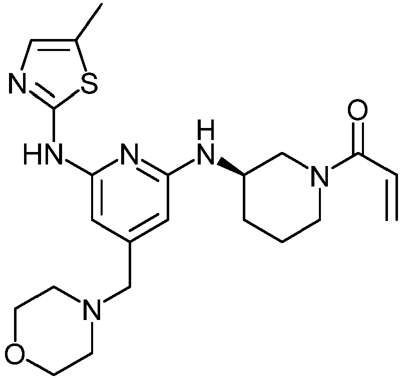
(3) 1-(3-((6-((5-methylthiazol-2-yl)amino)-4-(morpholinomethyl)pyridin-2-yl)amino)piperidin-1-yl )prop-2-en-1-one

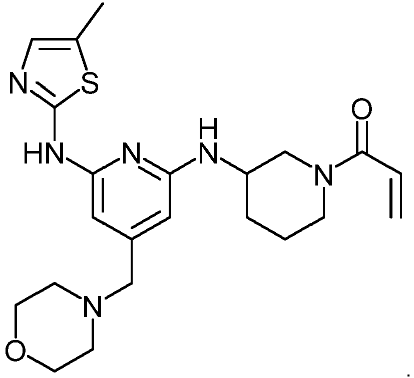

Hereinafter, the present disclosure will be described in detail for each step.

### (Step 1)

Step 1 is a step of preparing the compound represented by Chemical Formula 1-3 by reacting the compound represented by Chemical Formula 1-1 with the compound represented by Chemical Formula 1-2, and the reaction is an amine substitution reaction, which is carried out in the presence of a palladium catalyst and a base.

At this time, the compound represented by Chemical Formula 1-1 and the compound represented by Chemical Formula 1-2 may be used in a molar ratio of 1:0.1 to 1:2. Specifically, the compound represented by Chemical Formula 1-1 and the compound represented by Chemical Formula 1-2 may be used in a molar ratio of 1:0.5 to 1:1.7, 1:0.7 to 1:1.5, or 1:0.9 to 1:1.5.

In addition, the palladium catalyst may be a palladium(0) catalyst which is zero-valence palladium (Pd) in the compound, or a palladium(II) catalyst with a valence of +2. For example, as the palladium catalyst, at least one selected from the group consisting of tris(dibenzylideneacetone)dipalladium(0), tetrakis(triphenylphosphine) palladium(0), bis[tris(2-methylphenyl)phosphine]palladium and palladium(II) acetate can be used.

Further, the base used in the reaction of step 1 may be at least one selected from the group consisting of cesium carbonate, potassium carbonate, sodium carbonate, sodium tert-butoxide and potassium tert-butoxide. Among them, cesium carbonate is preferably used in terms of reaction rate and yield.

Further, the reaction may be carried out in the presence of a phosphine-based compound together with the palladium catalyst and the base. As the phosphine-based compound, at least one selected from the group consisting of 2,2'-bis(diphenylphosphino)-1,1'-binaphthyl, 4,5-bis(diphenylphosphino)-9,9-dimethyl xanthene, 2-(dicyclohexylphosphino)-3,6-dimethoxy-2,4',6'-triisopropyl-1,1'-biphenyl and dicyclohexylphosphino-2',6'-diisopropoxybiphenyl can be used.

In addition, as a solvent for the reaction, a solvent inactive for the amine substitution reaction can be used. For example, a solvent such as toluene, dioxane, dimethylformamide (DMF), butyl alcohol, or dimethylacetamide (DMAc) can be used, but toluene and dimethylacetamide (DMAC) are preferably used in terms of reaction rate and yield.

At this time, the solvent may be used in an amount (mL/g) of 10 to 30 times (volume), more specifically in an amount (mL/g) of 15 to 25 times (volume), relative to the weight of the compound represented by Chemical Formula 1-1.

Further, the reaction may be carried out at a temperature of 80 to 150°C for 3 hours to 15 hours. When the reaction is carried out under a lower temperature condition and/or for a shorter reaction time than the above-mentioned range, the reaction does not proceed sufficiently, and the production yield may be lowered. In addition, even if the reaction is carried out under a higher temperature condition and/or a longer reaction time than the above-mentioned range, the production yield is not substantially increased, which is undesirable in terms of process cost. More specifically, the reaction may be carried out at a temperature of 90 to 130°C for 4 hours to 12 hours, preferably 4 hours to 10 hours, more preferably 6 hours to 10 hours.

Further, when using a combination of the above-mentioned palladium catalyst, base, and phosphine-based compound, the reaction may be carried out in a conventionally known reactor without the use of a microwave reactor. Thereby, step 1) can be applied as a process for mass-producing the compound represented by Chemical Formula 1.

On the other hand, after the reaction is completed, without the step of purifying or isolating the prepared compound represented by Chemical Formula 1-3, the process up to step 1 and step 2 described hereinafter can be carried out in-situ in the same reaction vessel. As described above, since the step of purifying or isolating the compound is not required in step 1, the method for preparing the compound represented by Chemical Formula 1 including step 1 may be advantageous in terms of industrial mass production of the compound.

### (Step 2)

Step 2 is a step of preparing a compound represented by Chemical Formula 1-4 by reacting a compound represented by Chemical Formula 1-3 in the presence of an acid, which is a step of removing tert-butyloxycarbonyl group, which is a protecting group substituted on the piperidine ring of the compound represented by Chemical Formula 1-3, in the presence of an acid.

As the acid used in the reaction of step 2), hydrochloric acid, acetic acid, trichloroacetic acid, trifluoroacetic acid or the like can be used. Among them, hydrochloric acid is preferred in consideration of yield and cost.

As a solvent for the reaction, a solvent such as ethyl acetate, methyl alcohol, dioxane, or toluene can be used.

Further, the reaction may be carried out at room temperature for 30 minutes to 20 hours, preferably 30 minutes to 4 hours. The reaction of removing tert-butyloxycarbonyl group, which is a protecting group, corresponds to a reaction that easily occurs without additional heat treatment, and thus can be carried out at room temperature. However, if the reaction time is less than 30 minutes, the reaction may not proceed sufficiently, and if the reaction time exceeds 20 hours, the production yield is not substantially increased. Therefore, the above-mentioned reaction time is preferred.

On the other hand, after the reaction is completed, in order to purify the compound represented by Chemical Formula 1-4, if necessary, a step of crystallizing the reaction product can be further included. In the crystallizing step, the crystallized compound represented by Chemical Formula 1-4 is prepared by using a crystallization solvent in the reaction product. In other words, step 2) can be carried out through the steps of reacting the compound represented by Chemical Formula 1-3 in the presence of an acid, and then crystallizing the reaction product to prepare the compound represented by Chemical Formula 1-4.

Specifically, the crystallizing step may be carried out by a primary crystallization with water and a secondary crystallization with methanol. As described above, when the reaction product generated after the completion of the reaction of step 2) is crystallized sequentially with water and methanol, it is possible to effectively remove palladium catalyst, ligand, aminothiazole residue, etc., which are by-products remaining after the reaction. Thereby, a high-purity compound can be obtained in a high yield as compared to the step of purifying in the form of a slurry using a solvent such as ethyl acetate.

The primary crystallization step with water can be carried out by adding water to the reaction product and stirring the same at a temperature of about 0 to 5°C for 10 minutes to 2 hours. Further, the crystallization is preferably carried out in the presence of a base such as sodium hydroxide while maintaining the pH of the reaction product at 11 or more, or 12 or more. When crystals are formed under the above conditions, after completion of the reaction the reaction solution can be filtered under reduced pressure to obtain crystals.

At this time, water can be used in an amount (mL/g) of 5 to 30 times (volume), more specifically in an amount (mL/g) of 5 to 25 times (volume), relative to the weight of the compound represented by Chemical Formula 1-1.

Next, after the resulting crystal is dried, a secondary crystallization step with methanol can be carried out. The secondary crystallization step with methanol may be carried out by adding methanol to the resulting crystals and stirring the same at a temperature of about 50 to 80°C for 10 minutes to 2 hours.

At this time, methanol may be used in an amount (mL/g) of 5 to 40 times (volume), more specifically in an amount (mL/g) of 10 to 35 times (volume), relative to the weight of the compound represented by Chemical Formula 1-1.

### (Step 3)

Step 3 is a step of preparing a compound represented by Chemical Formula 1 by reacting a compound represented by Chemical Formula 1-4 with a compound represented by Chemical Formula 1-5. The reaction is an amidation reaction, which is preferably carried out in the presence of a base.

At this time, the compound represented by Chemical Formula 1-4 and the compound represented by Chemical Formula 1-5 can be used in a molar ratio of 1:0.5 to 1:2.0. Specifically, the compound represented by Chemical Formula 1-4 and the compound represented by Chemical Formula 1-5 can be used in a molar ratio of 1:0.5 to 1:1.5, 1:0.7 to 1:1.3, or 1:0.9 to 1:1.1.

As the base used in the reaction of step 3), at least one selected from the group consisting of potassium carbonate, sodium hydroxide, lithium hydroxide, potassium hydroxide, triethylamine, diisopropylamine, diisopropylethylamine, sodium hydrogen carbonate, potassium hydrogen carbonate, cesium carbonate, sodium carbonate, sodium methylate and potassium butyrate can be used. Among them, potassium carbonate, sodium carbonate, sodium hydrogen carbonate, or potassium hydrogen carbonate is preferably used in terms of completion of the reaction and generation of by-products.

As a solvent for this reaction, a mixed solvent of tetrahydrofuran (THF) and water can be used. At this time, the tetrahydrofuran can be used in an amount (mL/g) of 10 to 40 times (volume) relative to the weight of the compound represented by Chemical Formula 1-4, and the water can be used in an amount (mL/g) of 2 to 10 times (volume) relative to the weight of the compound represented by Chemical Formula 1-4.

On the other hand, the compound represented by Chemical Formula 1-5 can be added in a state of being mixed with N,N-diisopropylethylamine or triethylamine. Acryloyl chloride, which is the compound represented by Chemical Formula 1-5, has a small amount of hydrochloric acid remaining in the compound. Thereby, the hydrochloric acid participates in the reaction and generates, as a by-product, impurities (hereinafter, referred to as impurity C (ImpC)), such as a compound in which HCI is added to the double bond of the compound represented by Chemical Formula 1 and a compound in which HCI is added to the double bond of acrylamide, which causes a problem that the purity of the final compound is reduced. However, when the compound represented by Chemical Formula 1-5 is used in a solution state in which it is dissolved in a solvent such as tetrahydrofuran together with N,N-diisopropylethylamine (DIPEA), a small amount of hydrochloric acid remaining in the compound represented by Chemical Formula 1-5 can be removed in advance by N,N-diisopropylethylamine or triethylamine, and thus, the impurity C may not be generated.

At this time, in a solution in which the compound represented by Chemical Formula 1-5 and N,N-diisopropylethylamine are mixed, the N,N-diisopropylethylamine may be contained in an amount of 0.01 to 0.2 mole relative to 1 mole of the compound represented by Chemical Formula 1-5. Within the above range, hydrochloric acid in the compound represented by Chemical Formula 1-5 can be effectively removed.

Further, the reaction is carried out at a temperature of -10°C or more and less than 0°C. A dimer of the compound represented by Chemical Formula 1 (hereinafter, referred to as impurity B (ImpB)) may be prepared by the above reaction. When the reaction proceeds at a low temperature, it is possible to minimize the generation of such impurity B. Specifically, a reaction temperature of -10 to 0°C, more specifically, a reaction temperature of -8 to -3°C, is preferred in terms of impurity reduction and production yield. Therefore, it is preferable that all the reaction reagents such as reactants and organic solvents, which are used to suppress the increase in reaction temperature during the reaction, are used after being cooled to a temperature of 0°C or less.

On the other hand, after the reaction is completed, if necessary, at least one of the steps of extraction of the reaction product, purification by concentration under reduced pressure, and crystallization can be included, thereby isolating and purifying the compound represented by Chemical Formula 1. In order to prepare a high-purity compound, it is preferable to sequentially perform all of the steps of extracting, purifying, and crystallizing the reaction product.

Specifically, after the reaction of step 3 is completed, a step of extracting the reaction product using ethyl acetate can be further included. Specifically, the reaction product may be extracted using ethyl acetate and water. That is, the compound represented by Chemical Formula 1 may be extracted using ethyl acetate and water after completion of the reaction. When extracting in this way, impurities having a relative retention time of less than 1.0, which are isolated by high-performance liquid chromatography (HPLC), can be effectively removed with water layer, as compared to when extracting using a methylene chloride / water mixed solvent.

The extraction step with ethyl acetate may be then carried out at a pH of 6.5 to 7.5. If the pH is lower than 6.5, there is concern that the yield is lowered, and if the pH is higher than 7.5, the removal of impurities may be difficult. More specifically, the extraction step with ethyl acetate may be carried out in the range of pH 7.0 to 7.5.

In addition, step 3 may further include a step of dissolving the reaction product in tetrahydrofuran; a step of mixing a phosphoric acid-containing solution with the prepared solution; and a purification step of filtering the prepared mixture and then concentrating the filtrate under reduced pressure. Such step of concentration under reduced press is preferably carried out after the extraction step with ethyl acetate.

In other words, the purification step may include a step of dissolving the product extracted with ethyl acetate in tetrahydrofuran to prepare a solution; a step of mixing a phosphoric acid-containing solution with the solution to prepare a mixture in which a phosphate salt is produced; and a step of filtering the mixture in which the phosphate salt is produced, and then concentrating the filtrate under reduced pressure.

The phosphoric acid-containing solution is a solution in which phosphoric acid is dissolved in a tetrahydrofuran solvent, wherein the phosphoric acid may be dissolved in an amount of 0.05 to 0.5 moles relative to 1 mole of the compound represented by Chemical Formula 1-4. When a phosphoric acid-containing solution in the above-mentioned range is added to the product, a compound in which a compound represented by Chemical Formula 1 and a compound represented by Chemical Formula 1-4 produced during the reaction are bonded (hereinafter, referred to as impurity A (ImpA)) reacts with a phosphate anion (PO₄⁻³) to produce a phosphate salt. However, the produced phosphate salt can be easily removed by filtration, and thus, the content of impurity A in the final produced compound represented by Chemical Formula 1 may be lowered.

In addition, step 3 may further include a step of crystallizing the reaction product with ethyl acetate. This crystallization step is preferably carried out after the purification step.

In other words, the crystallization step can, after the purification step, be carried out by adding ethyl acetate to the product concentrated under reduced pressure and then stirring at a temperature of about 20 to 40°C for 30 minutes to 4 hours. When crystals are produced under the above conditions, the reaction solution can be dried under reduced pressure after completion of the reaction, thereby obtaining a compound represented by Chemical Formula 1-1 which is a final product.

That is, when all of the steps of extracting, purifying, and crystallizing the reaction product of step 3 are sequentially carried out to prepare the compound represented by Chemical Formula 1, step 3 can be carried out throughn the steps of:
a step of reacting the compound represented by Chemical Formula 1-4 with the compound represented by Chemical Formula 1-5 in the presence of a base;
a step of extracting the reaction product using ethyl acetate;
a step of dissolving the product extracted with ethyl acetate in tetrahydrofuran to prepare a solution, a step of mixing a phosphoric acid-containing solution with the solution to prepare a mixture in which a phosphate salt is produced, and a step of filtering the mixture in which the phosphate salt is produced and then concentrating the filtrate under reduced pressure; and
a step of crystallizing the product concentrated under reduced pressure with ethyl acetate.

When additional extraction, purification and crystallization steps are carried out after the reaction in this way, it is possible to prepare a high-purity compound represented by Chemical Formula 1 in which the content of impurity (RRT <1.0) having an RRT of less than 1.0, impurity A (ImpA), impurity B (ImpB) and impurity C (ImpC) is significantly low, which can be confirmed by comparing Examples and Reference Examples described hereinafter.

### [ADVANTAGEOUS EFFECTS]

As described above, the preparation method according to the present disclosure has an advantage that a heterocyclic amine derivative having a reduced impurity content can be prepared in a high yield.

### [DETAILED DESCRIPTION OF THE EMBODIMENTS]

Below, the present disclosure will be described in more detail with reference to the following Examples. However, these examples are for illustrative purposes only, and the scope of the present disclosure is not limited thereby. In addition, the equivalent (eq) in the following may be understood to mean the number of moles relative to the starting material in the reaction proceeded at each step.

### Preparation Example: Preparation of Compound represented by Chemical Formula 1-1 ((S)-tert-butyl 3-((6-chloro-4-(morpholinomethyl)pyridin-2-yl)amino)piperidine-1-carboxylate)

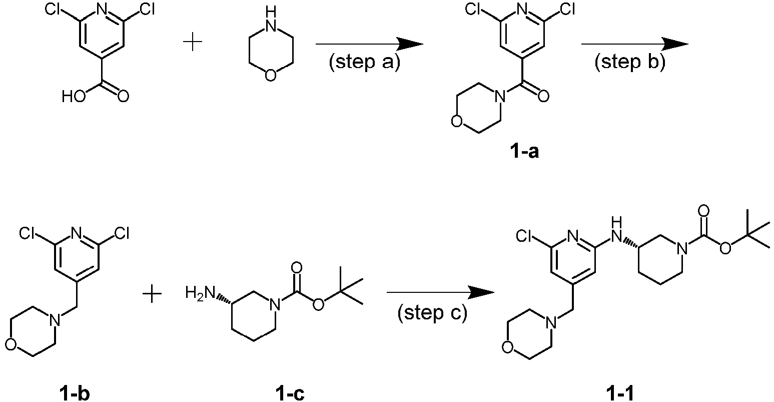

### (Step a)

2,6-dichloroisonicotinic acid (10.0 g, 1.0 eq) was dissolved in dimethylformamide (100.0 mL), and then 1,1-carbonyldiimidazole (1.0 g, 1.2 eq) was added thereto. The mixture was stirred at room temperature (25-30°C) under nitrogen gas for 1 hour, then morpholine (5.4 mL, 1.2 eq) was added and stirred at the same temperature for 2 hours to complete the reaction. Ethyl acetate (200.0 mL) and water (200.0 mL) were added and extracted, and the water layer was re-extracted three times using ethyl acetate (200.0 mL). The ethyl acetate layer was dried over anhydrous sodium sulfate, and then concentrated under reduced pressure. The resulting residue was purified by column chromatography (ethyl acetate:hexane = 1:5) to obtain the compound represented by Chemical Formula 1-a, (2,6-dichloropyridin-4-yl)(morpholino)methanone (13.0 g, 93.0%).

¹H-NMR (500 MHz, CDCl₃): 7.27(s, 2H), 3.77(m, 4H) 3.65(m, 2H), 3.37(m, 2H)

### (Step b)

The compound represented by Chemical Formula 1-a (10.0 g, 1.0 eq) obtained in step a was dissolved in dichloromethane (100.0 mL), and then cooled to 0 to 10°C under nitrogen gas. 1M borane-tetrahydrofuran (115.0 mL, 3.0 eq) was slowly added dropwise thereto, and then stirred at room temperature for 12 hours to complete the reaction. The reaction solution was cooled to 0~10°C, and then 6N-hydrochloric acid aqueous solution (256.0 mL, 20.0 eq) was slowly added dropwise and then stirred at the same temperature for 1 hour. The pH was adjusted to 9 to 12 using a 10N aqueous sodium hydroxide solution, and then extracted twice with dichloromethane. The dichloromethane layer was isolated, dried over anhydrous sodium sulfate, and then concentrated under reduced pressure. The resulting residue was purified by column chromatography (ethyl acetate: hexane = 1:1) to obtain the compound represented by Chemical Formula 1-b, 4-((2,6-dichloropyridin-4-yl)methyl)morpholine (8.1 g, yield: 90.0%).

¹H-NMR (500 MHz, CDCl₃): 7.27(s, 2H), 3.72(m, 4H) 3.46(s, 2H), 2.45(m, 4H)

### (Step c)

The compound represented by Chemical Formula 1-b (1.0 g, 1.0 eq) obtained in step b was dissolved in 1,4-dioxane (10.0 mL), and then tris(dibenzylideneacetone)dipalladium(0) (465.8 mg , 0.2 eq) and Xantphos (1.5 g, 0.4 eq) were added thereto. (S)-tert-butyl 3-aminopiperidine-1-carboxylate (780.0 *µ*ℓ, 1.0 eq), which is a compound represented by Chemical Formula 1-c, was added, and then sodium carbonate (1.3 g, 3.0 eq) was added, and the mixture was then refluxed for 12 hours to complete the reaction. After cooling to 30°C or less, water (20.0 mL) and ethyl acetate (20.0 mL) were added thereto, and then the layers were isolated. The ethyl acetate layer was dried over anhydrous sodium sulfate, and then concentrated under reduced pressure. The resulting residue was purified by column chromatography (ethyl acetate: hexane = 1:1) to obtain the compound represented by Chemical Formula 1-1, (S)-tert-butyl 3-((6-chloro-4-(morpholnomethyl)pyridin-2-yl)amino)piperidine-1-carboxylate (900.0 mg, yield: 54.1%).

¹H-NMR (500 MHz, CDCl₃): 6.63(s, 1H), 6.31(s, 1H), 4.66-4.65(m,1H), 3.84-3.82(m, 1H) 3.75-3.70(m, 5H), 3.59-3.54(m, 1H), 3.37(s, 2H), 3.27-3.23(m, 2H), 2.46(t, 4H), 1.98-1.94(m, 1H), 1.73(s, 1H), 1.63-1.56(m, 2H), 1.50(s, 9H)

### Example: Preparation of (S)-1-(3-((6-((5-methylthiazol-2-yl)amino)-4-(morpholinomethyl)pyridin-2-yl)amino)piperidin-1-yl) prop-2-en-1-one

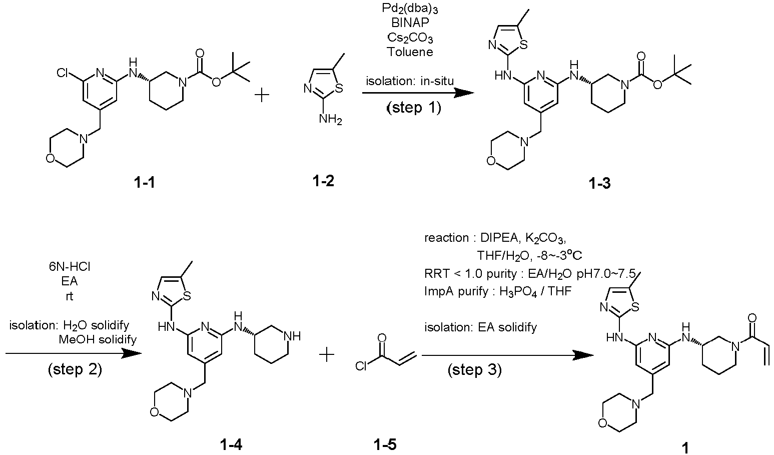

### (Step 1)

The compound represented by Chemical Formula 1-1 (50.0 g, 1.0 eq) obtained in Preparation Example, the compound represented by Chemical Formula 1-2 (16.7 g, 1.2 eq), tris(dibenzylideneacetone)dipalladium(0) (11.1 g, 0.1 eq), 2,2'-bis(diphenylphosphino)-1,1'-binaphthyl (BINAP) (30.3 g, 0.4 eq), cesium carbonate (118.9 g, 3.0 eq) and 1000 mL of toluene with a volume of 20 times relative to the weight of the compound represented by Chemical Formula 1-1 were added to a flask, and the mixture was stirred at room temperature for 10 minutes. After raising the temperature inside the flask to 110°C, the reaction mixture was stirred at a temperature range of 105~111°C for 8 hours. The resulting residue was filtered through Celite to remove the catalyst and the produced inorganic salt. Then, the organic layer was concentrated under reduced pressure, and 250 mL of purified water with a volume of 5 times relative to the weight of the compound represented by Chemical Formula 1-1 and 1000 mL of ethyl acetic acid with a volume of 20 times relative to the weight of the compound represented by Chemical Formula 1-1 were added thereto and the organic layer was extracted. The aqueous layer generated here was discarded. Then, the organic layer was concentrated under reduced pressure at 40~45°C to prepare the compound represented by Chemical Formula 1-3, which was used subsequent to step 2 below without further purification.

¹H-NMR (500 MHz, DMSO): 10.51(s, 1H), 6.93(s, 1H) 6.40(s, 1H), 6.06(s, 1H), 5.98(s, 1H), 4.01(s, 2H), 3.55(s, 5H), 3.21(s, 4H), 2.32(s, 4H), 2.24(s, 3H), 1.97(s, 1H), 1.72(s, 1H), 1.32(br s, 2H),1.15(s, 9H)

### (Step 2)

Ethyl acetate (EA) (500 mL) and 6N-HCI (250 mL, 5.0 eq) were added to the compound represented by Chemical Formula 1-3 prepared in step 1, and then the mixture was stirred at room temperature for 2 hours to complete the reaction.

### Crystallization step with water and methanol

Then, the water layer was extracted, and 1000 mL of purified water (an amount (mL/g) of 20 times (volume) relative to the weight of the compound represented by Chemical Formula 1-1) was added to the organic layer, and subjected to secondary extraction. The water layers obtained in the 1st and 2nd were combined, the internal temperature was maintained at 0~5°C by using an ice-bath, and 700 mL of 4N-NaOH (an amount (mL/g) of 14 times (volume) relative to the weight of the compound represented by Chemical Formula 1-1) was added for about 30 minutes, and the mixture was stirred for 30 minutes while maintaining the pH of 12 or higher. The resulting crystals were filtered under reduced pressure, and the filtrate was washed with 500 mL of purified water (an amount (mL/g) of 10 times (volume) relative to the weight of the compound represented by Chemical Formula 1-1) and 500 mL of hexane (an amount (mL/g) of 10 times (volume) relative to the weight of the compound represented by Chemical Formula 1-1). At this time, the washed filtrate was placed in a dryer, and then vacuum dried at a temperature of 40 ~45°C for 12 hours or more, and then 1.65 L of methanol (an amount (mL/g) of 33 times (volume) relative to the weight of the compound represented by Chemical Formula 1-1) was added thereto, and the mixture was stirred at an internal temperature of 65°C for about 30 minutes and then cooled to room temperature. The resulting crystals were filtered under reduced pressure, the resulting filtrate was placed in a dryer, and then vacuum dried at a temperature of 40-45°C for 12 hours or more to obtain 28.5 g of the compound represented by Chemical Formula 1-4 (yield: 60.3% (including steps 1 and 2)).

¹**H-NMR** (500 MHz, DMSO): 10.47 (s, 1H), 6.93(s, 1H) 6.28(d, 1H), 6.02(s, 1H), 5.94(s, 1H), 3.91(s, 1H), 3.56 (s, 4H), 3.20 (s, 2H), 3.06 (d, 1H), 2.79 (d, 1H), 2.44-2.42 (m, 1H), 2.37-2.34 (m, 1H), 2.32(s, 3H), 2.28(s, 4H), 2.03-2.01(m, 2H), 1.63-1.60(m, 1H), 1.44-1.42 (m, 1H), 1.30-1.28 (m, 1H)

### (Step 3)

To the compound represented by Chemical Formula 1-4 (5.0 g, 1.0 eq) prepared in step 2, 90.0 mL of THF (an amount (mL/g) of 18 times (volume) relative to the weight of the compound represented by Chemical Formula 1-4) and 20.0 mL of H₂O (an amount (mL/g) of 4 times (volume) relative to the weight of the compound represented by Chemical Formula 1-4) were added to prepare a solution at room temperature. Then, the internal temperature was cooled to -8 ~ -3°C using an external temperature -8~-5°C bath (mixture of ice, H₂O, and MeOH), and then K₂CO₃ (2.6 g, 1.5 eq) was added and dissolved. Here, to 5 mL of THF cooled to -8~-3°C, a mixed solution of N,N-diisopropylethylamine (DIPEA) (111.5 *µ*ℓ, 0.05 eq) and the compound represented by Chemical Formula 1-5 (1.1 mL, 1.05 eq) was added dropwise at an internal temperature of -8~-3°C. The resulting mixture was stirred at -8~-3 °C for 0.5 hours to complete the reaction.

### Extraction step with ethyl acetate

To the reaction vessel where reactions were completed, 100.0 mL of ethyl acetate at a temperature of -8 ~ -3°C (an amount (mL/g) of 20 times (volume) relative to the weight of the compound represented by Chemical Formula 1-4) and 50.0 mL of saturated NH₄Cl aqueous solution (an amount (mL/g) of 10 times (volume) relative to the weight of the compound represented by Chemical Formula 1-4) were added, and the layers were isolated and only the organic layer was isolated. Then, 50.0 mL of ethyl acetate at a temperature of -8 ~ -3°C (an amount (mL/g) of 10 times (volume) relative to the weight of the compound represented by Chemical Formula 1-4) was added to the aqueous layer, and re-extracted. Then, only the organic layer was collected and concentrated under reduced pressure at an external temperature of 35°C. 100.0 mL of ethyl acetate at a temperature of -8 ~ -3°C (an amount (mL/g) of 20 times (volume) relative to the weight of the compound represented by Chemical Formula 1-4) and 50.0 mL of H₂O at a temperature of 0 ~ 5°C (an amount (mL/g) of 10 times (volume) relative to the weight of the compound represented by Chemical Formula 1-4) were added thereto, the pH was adjusted to 7.0-7.5 with a 1N-HCI solution, and the layers were isolated twice. The isolated organic layer was collected, dried over Na₂SO₄, and then concentrated under reduced pressure at an external temperature of 35°C.

### Purification step with phosphoric acid (H₃PO₄)

90.0 mL of THF at a temperature of -8~-3°C (an amount (mL/g) of 18 times (volume) relative to the weight of the compound represented by Chemical Formula 1-4) was added thereto and made to dissolve. Then, a solution of H₃PO₄ (9.3 g, 0.095 eq) dissolved in 5.0 mL of THF at a temperature of -8~-3°C was added dropwise at a temperature of -8~-3°C, and then the mixture was stirred for 30 minutes. The mixture was filtered through Celite to remove salts, and a solution of H₃PO₄ (8.3 g, 0.085 eq) dissolved in THF 5.0 mL at a temperature of -8~-3°C was added dropwise to the filtrate, and the solution was stirred for 30 minutes at -8~-3 °C. The resulting residue was filtered through Celite to remove salts, and the filtrate was concentrated under reduced pressure at an external temperature of 35°C. 25.0 mL of methylene chloride (MC) at a temperature of -8~-3°C (an amount (mL/g) of volume 5 times (volume) relative to the weight of the compound represented by Chemical Formula 1-4), and 10.0 mL of H₂O at a temperature of 0 to 5°C (an amount (mL/g) of 2 times (volume) relative to the weight of the compound represented by Chemical Formula 1-4) were added thereto to adjust the pH to 9.0 ~ 9.5, and then the organic layer was isolated. The isolated organic layer was dried with Na₂SO₄ and then concentrated under reduced pressure at an external temperature of 35°C.

### Crystallization step with ethyl acetate

75.0 mL of ethyl acetate (an amount (mL/g) of 15 times (volume) relative to the weight of the compound represented by Chemical Formula 1-4) was added thereto, and then, when crystals were formed, they were stirred at 20~30°C for 2 hours and filtered. The filtrate was dried under reduced pressure at room temperature for 12 hours to obtain 3.4 g of the compound represented by Chemical Formula 1, (S)-1-(3-((6-((5-methylthiazol-2-yl)amino)-4-(morpholinomethyl)pyridin-2-yl)amino)piperidin-1-yl) prop-2-en-1-one (yield: 60.0%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Reference Example 1

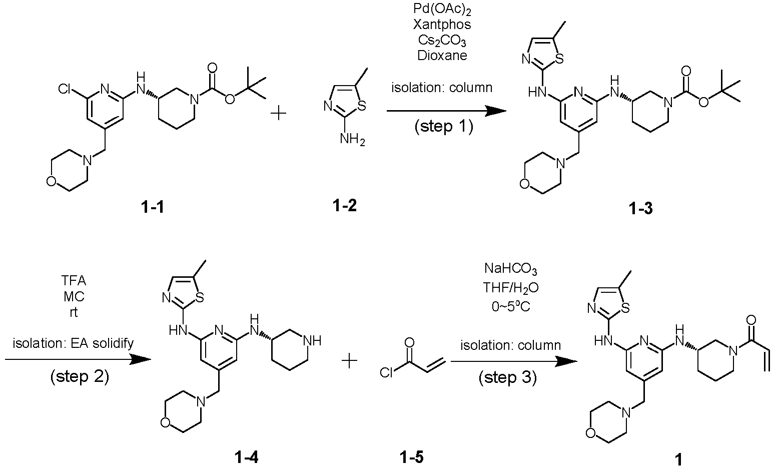

### (Step 1)

The compound represented by Chemical Formula 1-1 (730.0 mg, 1.0 eq) obtained in Preparation Example was dissolved in 1,4-dioxane (14.0 mL). Palladium acetate (40.0 mg, 0.1 eq), Xantphos (204.7 mg, 0.2 eq), the compound represented by Chemical Formula 1-2 (203.6 mg, 1.0 eq) and cesium carbonate (1.7 g, 3.0 eq) were sequentially added. The reaction was carried out in a microwave reactor at 150°C for 30 minutes. The reaction mixture was cooled to 30°C or less, and then water (15.0 mL) and ethyl acetate (15.0 mL) were added thereto, and then the layers were isolated. The ethyl acetate layer was dried over anhydrous sodium sulfate, and then concentrated under reduced pressure. The resulting residue was purified by column chromatography (EA 100%) to obtain 564.0 mg of the compound represented by Chemical Formula 1-3 (yield: 65.0%).

¹H-NMR (500 MHz, DMSO): 10.51(s, 1H), 6.93(s, 1H) 6.40(s, 1H), 6.06(s, 1H), 5.98(s, 1H), 4.01(s, 2H), 3.55(s, 5H), 3.21(s, 4H), 2.32(s, 4H), 2.24(s, 3H), 1.97(s, 1H), 1.72(s, 1H), 1.32(br s, 2H),1.15(s, 9H)

### (Step 2)

The compound represented by Chemical Formula 1-3 (500.0 mg, 1.0 eq) obtained in step 1 was dissolved in dichloromethane (10.0 mL), and then cooled to 0-10°C. Trichloroacetic acid (1.6 mL, 20.0 eq) was slowly added dropwise thereto, and then the mixture was stirred for 1 hour. Then, the pH was adjusted to 9-12 using a 12N-sodium hydroxide aqueous solution, and then the isolated dichloromethane layer was dried over anhydrous sodium sulfate, and then concentrated under reduced pressure. ethyl acetate (10.0 mL) was added to the resulting residue to form crystals for 30 minutes. The resulting crystals were filtered and then dried to obtain 357.5 mg of the compound represented by Chemical Formula 1-4 (yield: 90.0%).

¹H-NMR (500 MHz, DMSO): 10.47 (s, 1H), 6.93(s, 1H) 6.28(d, 1H), 6.02(s, 1H), 5.94(s, 1H), 3.91(s, 1H), 3.56 (s, 4H), 3.20 (s, 2H), 3.06 (d, 1H), 2.79 (d, 1H), 2.44-2.42 (m, 1H), 2.37-2.34 (m, 1H), 2.32(s, 3H), 2.28(s, 4H), 2.03-2.01(m, 2H), 1.63-1.60(m, 1H), 1.44-1.42 (m, 1H), 1.30-1.28 (m, 1H)

### (Step 3)

The compound represented by Chemical Formula 1-4 (350.0 mg, 1.0 eq) obtained in step 2 was dissolved in tetrahydrofuran (7.0 mL), to which water (7.0 mL) was added, sodium bicarbonate (226.8 mg, 3.0 eq) was added, and then was cooled to 0~10°C. The compound represented by Chemical Formula 1-5 (73.1 *µ*ℓ, 1.0 eq) was slowly added dropwise thereto, and then the mixture was stirred for 30 minutes to complete the reaction. This was layer-isolated using dichloromethane, dried over anhydrous sodium sulfate, and concentrated under reduced pressure. The resulting residue was purified by column chromatography (dichloromethane:methanol = 15:1) to obtain 318.0 mg of the compound represented by Chemical Formula 1 (yield: 60.0%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Reference Example 2

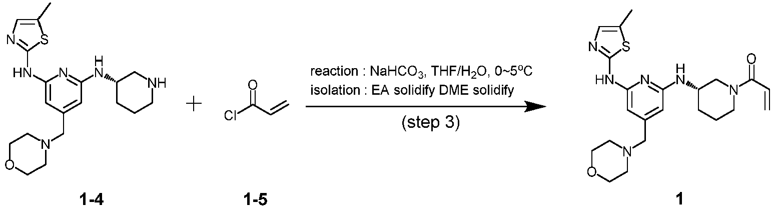

### (Steps 1 and 2)

The compound represented by Formula 1-4 was prepared by using the same methods as in steps 1 and 2 prepared in Example.

### (Step 3)

The compound represented by Chemical Formula 1-4 (350.0 mg, 1.0 eq) obtained in step 2 was dissolved in tetrahydrofuran (7.0 mL), to which water (7.0 mL) was added, sodium bicarbonate (226.8 mg, 3.0 eq) was added, and then cooled to 0-10°C. The compound represented by Chemical Formula 1-5 (73.1 *µ*ℓ, 1.0 eq) was slowly added dropwise thereto, and the mixture was stirred for 30 minutes to complete the reaction. This was layer-isolated using dichloromethane, dried over anhydrous sodium sulfate, and concentrated under reduced pressure. Ethyl acetate was added to the resulting residue by an amount (mL/g) of 20 times (volume) relative to the weight of the residue, and then stirred at room temperature for 3 hours to form crystals. The resulting crystals were filtered, dried under reduced pressure at room temperature, and dimethoxyethane was added thereto by an amount (mL/g) of 15 times (volume) relative to the weight of the crystals, then dissolved under reflux, and slowly cooled to room temperature, and then stirred for 2 hours to form crystals. This was filtered and dried under reduced pressure at room temperature to obtain 0.12 g of the compound represented by Chemical Formula 1 as the 6th material (yield: 30.0%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Reference Example 3

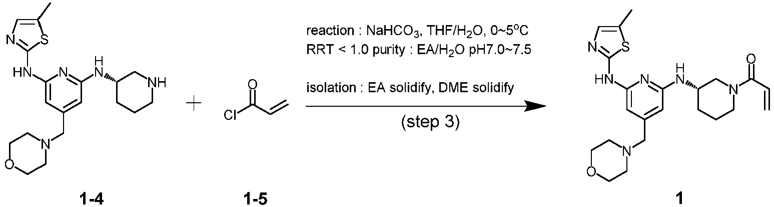

### (Steps 1 and 2)

The compound represented by Chemical Formula 1-4 was prepared by using the same methods as in steps 1 and 2 prepared in Example.

### (Step 3)

To the compound represented by Chemical Formula 1-4 (2.34 g, 1.0 eq) obtained in step 2, 46.8 mL of THF (an amount (mL/g) of 20 times (volume) relative to the weight of the compound represented by Chemical Formula 1-4) and 9.4 mL of H₂O (an amount (mL/g) of 4 times (volume) relative to the weight of the compound represented by Chemical Formula 1-4) were added to prepare a solution at room temperature. Then, the internal temperature was cooled to 0~5°C using an external temperature 0-5°C bath (mixture of ice and H₂O), and then NaHCO₃ (1.51 g, 3.0 eq) was added and dissolved. A solution in which the compound represented by Chemical Formula 1-5 (0.51 mL, 1.05 eq) was mixed was added dropwise thereto at an internal temperature of 0~5°C. This was stirred at 0~5°C for 0.5 hours to complete the reaction.

### Extraction step with ethyl acetate

To the reaction vessel where reactions were completed, 46.8 mL of ethyl acetate (an amount (mL/g) of 20 times (volume) relative to the weight of the compound represented by Chemical Formula 1-4) and 23.4 mL of H₂O (an amount (mL/g) of 10 times (volume) relative to the weight of the compound represented by Chemical Formula 1-4) were added, and then layer-isolated to isolate only the organic layer. Then, 23.4 mL of ethyl acetate (an amount (mL/g) of 10 times (volume) relative to the weight of the compound represented by Chemical Formula 1-4) was added to the aqueous layer and re-extracted, and then only the organic layer was collected and concentrated under reduced pressure at an external temperature of 40°C. 46.8 mL of ethyl acetate (an amount (mL/g) of 20 times (volume) relative to the weight of the compound represented by Chemical Formula 1-4) and 23.4 mL of H₂O (an amount (mL/g) of 10 times (volume) relative to the weight of the compound represented by Chemical Formula 1-4) were added, the pH was adjusted to 7.0-7.5 with 1N-HCI solution, and then the layers were isolated twice. The isolated organic layer was collected, dried over Na₂SO₄, and then concentrated under reduced pressure at an external temperature of 40°C.

### Crystallization step with ethyl acetate

46.8 mL of ethyl acetate (an amount (mL/g) of 20 times (volume) relative to the weight of the compound represented by Chemical Formula 1-4) was added thereto, and then, when crystals were formed, they were stirred at 20~30°C for 2 hours and filtered. The filtrate was dried under reduced pressure at room temperature for 12 hours to obtain 1.75 g of the compound represented by Chemical Formula 1, (S)-1-(3-((6-((5-methylthiazol-2-yl)amino)-4-(morpholinomethyl)pyridin-2-yl)amino)piperidin-1-yl)prop-2-en-1-one (yield: 68.9%).

### Crystallization step with dimethoxyethane

7.5 mL of dimethoxyethane (an amount (mL/g) of 15 times (volume) relative to the weight of the crystals with ethyl acetate) was added to 0.5 g of the crystals with ethyl acetate, and then dissolved at 50-60°C, and then naturally cooled to 20~30°C to form crystals. After crystals were formed, they were stirred at 20~30°C for 2 hours and filtered. The filtrate was dried under reduced pressure at room temperature for 12 hours to obtain 0.25 g of the compound represented by Chemical Formula 1, (S)-1-(3-((6-((5-methylthiazol-2-yl)amino)-4-(morpholinomethyl)pyridin-2-yl)amino)piperidin-1-yl)prop-2-en-1-one (yield: 35%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Reference Example 4

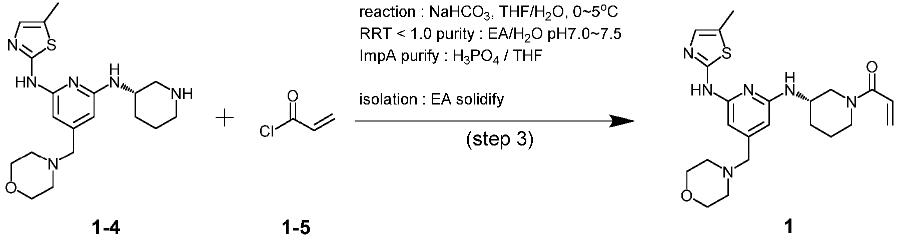

### (Steps 1 and 2)

The compound represented by Formula 1-4 was prepared by using the same methods as in steps 1 and 2 prepared in Example.

### (Step 3)

To the compound represented by Chemical Formula 1-4 (288.0 g, 1.0 eq) prepared in step 2, 5760.0 mL of THF (an amount (mL/g) of 20 times (volume) relative to the weight of the compound represented by Chemical Formula 1-4) and 1152.0 mL of H₂O (an amount (mL/g) of 4 times (volume) relative to the weight of the compound represented by Chemical Formula 1-4) were added to prepare a solution at room temperature. Then, the internal temperature was cooled to 0~5°C using an external temperature 0-5°C bath (mixture of ice and H₂O), and then NaHCO₃ (186.0 g, 3.0 eq) was added and dissolved. A solution in which the compound represented by Chemical Formula 1-5 (63.0 mL, 1.05 eq) was mixed was added dropwise thereto at an internal temperature of 0~5°C. This was stirred at 0~5°C for 0.5 hours to complete the reaction.

### Extraction step with ethyl acetate

To the reaction vessel where reactions were completed, 5760.0 mL of ethyl acetate (an amount (mL/g) of 20 times (volume) relative to the weight of the compound represented by Chemical Formula 1-4) and 2880.0 mL of H₂O (an amount (mL/g) of 10 times (volume) relative to the weight of the compound represented by Chemical Formula 1-4) were added, and then layer-isolated to isolate only the organic layer. Then, 1440.0 mL of ethyl acetate (an amount (mL/g) of 5 times (volume) relative to the weight of the compound represented by Chemical Formula 1-4) was added to the aqueous layer, re-extracted, and only the organic layer was collected and concentrated under reduced pressure at an external temperature of 40°C. 5760.0 mL of ethyl acetate (an amount (mL/g) of 20 times (volume) relative to the weight of the compound represented by Chemical Formula 1-4) and 2880.0 mL of H₂O (an amount (mL/g) of 10 times (volume) relative to the weight of the compound represented by Chemical Formula 1-4) were added, the pH was adjusted to 7.0-7.5 with 1N-HCI solution, and then the layers were isolated twice. The isolated organic layer was collected, dried over Na₂SO₄, and then concentrated under reduced pressure at an external temperature of 40°C.

### Purification step with phosphoric acid (H₃PO₄)

5184.0 mL of THF (an amount (mL/g) of 18 times (volume) relative to the weight of the compound represented by Chemical Formula 1-4) was added thereto and made to dissolve. Then, a solution of H₃PO₄ (6.8 g, 0.095 eq) dissolved in 288.0 mL of THF was added dropwise thereto, and then stirred for 30 minutes. The mixture was filtered through Celite to remove salts, and then a solution of H₃PO₄ (6.1 g, 0.085 eq) dissolved in 288.0 mL of THF was added dropwise to the filtrate, and then stirred for 30 minutes. The resulting residue was filtered through Celite to remove salts, and then concentrated under reduced pressure at an external temperature of 40°C.

### Crystallization step with ethyl acetate

5760.0 mL of ethyl acetate (an amount (mL/g) of 20 times (volume) relative to the weight of the compound represented by Chemical Formula 1-4) was added thereto, and then, when crystals were formed, they were stirred at 20~30°C for 2 hours and filtered. The filtrate was dried under reduced pressure at room temperature for 12 hours to obtain 195.5 g of the compound represented by Chemical Formula 1, (S)-1-(3-((6-((5-methylthiazol-2-yl)amino)-4-(morpholinomethyl)pyridin-2-yl)amino)piperidin-1- yl)prop-2-en-1-one (yield: 60.0%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Reference Example 5

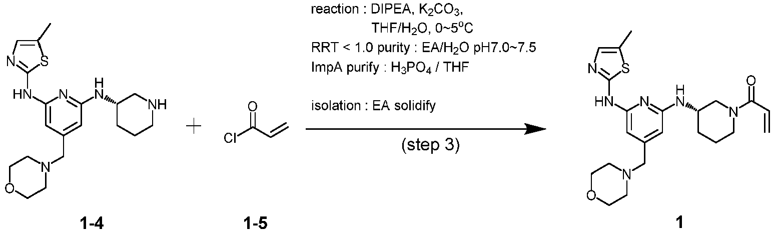

### (Steps 1 and 2)

The compound represented by Formula 1-4 was prepared by using the same methods as in steps 1 and 2 prepared in Example.

### (Step 3)

To the compound represented by Chemical Formula 1-4 (15.0 g, 1.0 eq) prepared in step 2, 270.0 mL of THF (an amount (mL/g) of 18 times (volume) relative to the weight of the compound represented by Chemical Formula 1-4) and 60.0 mL of H₂O (an amount (mL/g) of 4 times (volume) relative to the weight of the compound represented by Chemical Formula 1-4) were added to prepare a solution at room temperature. Then, the internal temperature was cooled to 0~5°C using an external temperature 0~5°C bath (mixture of ice and H₂O), and then K₂CO₃ (8.0 g, 1.5 eq) was added and dissolved. Here, to THF cooled to 0~5°C, a mixed solution of N,N-diisopropylethylamine (DIPEA) (334.4 *µ*ℓ, 0.05 eq) and the compound represented by Chemical Formula 1-5 (3.3 mL, 1.05 eq) was added dropwise at an internal temperature of 0~5°C. This was stirred at 0~5°C for 0.5 hours to complete the reaction.

### Extraction step with ethyl acetate

To the reaction vessel where reactions were completed, 300.0 mL of ethyl acetate at a temperature of 0-5°C (an amount (mL/g) of 20 times (volume) relative to the weight of the compound represented by Chemical Formula 1-4) and 150.0 mL of H₂O at a temperature of 0-5°C (an amount (mL/g) of 10 times (volume) relative to the weight of the compound represented by Chemical Formula 1-4) were added, and then layer-isolated to isolate only the organic layer. Then, 75.0 mL of ethyl acetate (an amount (mL/g) of 5 times (volume) relative to the weight of the compound represented by Chemical Formula 1-4) was added to the aqueous layer, re-extracted, and only the organic layer was collected and concentrated under reduced pressure at an external temperature of 40°C. 300.0 mL of ethyl acetate at a temperature of 0-5°C (an amount (mL/g) of 20 times (volume) relative to the weight of the compound represented by Chemical Formula 1-4) and 150.0 mL of H₂O (an amount (mL/g) of 10 times (volume) relative to the weight of the compound represented by Chemical Formula 1-4) were added thereto, the pH was adjusted to 7.0-7.5 with 1N-HCI solution, and then the layers were isolated twice. The isolated organic layer was collected, dried over Na₂SO₄, and then concentrated under reduced pressure at an external temperature of 35°C.

### Purification step with phosphoric acid (H₃PO₄)

270.0 mL of THF at 0~5°C (an amount (mL/g) of 18 times (volume) relative to the weight of the compound represented by Chemical Formula 1-4) was added thereto and dissolved. Then, a solution of H₃PO₄ (0.36 g, 0.095 eq) dissolved in 15.0 mL of THF was added dropwise, and then the mixture was stirred for 30 minutes. The mixture was filtered through Celite to remove salts, and a solution of H₃PO₄ (0.32 g, 0.085 eq) dissolved in 15.0 mL of THF was added dropwise to the filtrate, and the solution was stirred for 30 minutes. The resulting residue was filtered through Celite to remove salts, and then concentrated under reduced pressure at an external temperature of 35°C.

### Crystallization step with ethyl acetate

225.0 mL of ethyl acetate (an amount (mL/g) of 15 times (volume) relative to the weight of the compound represented by Chemical Formula 1-4) was added thereto, and then, when crystals were formed, they were stirred at 20~30°C for 2 hours and filtered. The filtrate was dried under reduced pressure at room temperature for 12 hours to obtain 10.2 g of the compound represented by Chemical Formula 1, (S)-1-(3-((6-((5-methylthiazol-2-yl)amino)-4-(morpholinomethyl)pyridin-2-yl)amino)piperidin-1-yl)prop-2-en-1-one (yield: 60.0%).

¹H NMR(500 MHz, DMSO): 10.57(m, 1H), 6.91-6.90(m, 1H), 6.80-6.85(m, 0.5H), 6.70-6.40(m, 1.5H), 6.10-5.96(m, 3H), 5.65-5.63(d, 0.5H), 5.42-5.40(d, 0.5H), 4.42-4.40(m, 0.5H), 4.10-4.0(m, 1H), 3.90-3.87(m, 1.5H), 3.56(m, 4H), 3.20(s, 2H), 3.14-3.10(m, 1H), 2.68-2.63(m, 0.5H), 2.32(m, 4H), 2.19(s, 3H), 1.90-2.0(m, 1H), 1.80(m, 1H), 1.50-1.40(m, 2.5H)

### Inhibitory Activity Against BTK and ITK

Inhibitory activities against BTK and ITK were measured for the compound represented by Chemical Formula 1 prepared in the above Examples as follows.

The inhibitory activities against BTK were evaluated using 'ADP-Glo^{™} + BTK Kinase enzyme system' kit (Promega Corporation). In a white 96-well plate, 10 *µ*ℓ of BTK enzyme prepared so as to have a final concentration of 10 ng/mℓ was mixed with 5 *µ*ℓ of compounds having a final concentration of 1 uM in the case of evaluating a single concentration of compound and a concentration of 1000, 200, 40, 8, 1.6 and 0.32 nM in the case of IC₅₀ evaluation, and then reacted at room temperature for 15 minutes. 5 *µ*ℓ of substrate and 5 *µ*ℓ of ATP prepared so as to have a final concentration of 10 uM were added to the plate on which reactions were completed, and then allowed to react at 30°C for 1 hour. All wells of the plate on which reactions were completed were treated with 25 *µ*ℓ of ADP-Glo^{™} reagent and allowed to react at 30°C for 40 minutes. After that, all wells were treated with 50 *µ*ℓ of kinase detection buffer, and then reacted for 30 minutes under light shielding conditions. For the plate on which all reactions were completed, luminescence was measured and the results were calculated. Evaluation was carried out in duplicate, and negative control and positive control were calculated depending on whether or not the enzyme was added without treatment of the compound. The IC₅₀ was calculated based on the calculated values.

In addition, the inhibitory activity against ITK was evaluated using 'ADP-Glo^{™} + ITK Kinase enzyme system' kit (Promega Corporation). In a white 96-well plate, 10 *µ*ℓ of ITK enzyme prepared so as to have a final concentration of 4 ng/mℓ was mixed with 5 *µ*ℓ of compounds having a final concentration of 1 uM in the case of evaluating a single concentration of compound and a concentration of 1000, 200, 40, 8, 1.6 and 0.32 nM in the case of IC₅₀ evaluation, and then reacted at room temperature for 15 minutes. To the plate on which reactions were completed, 5 ul of substrate and 5 *µ*ℓ of ATP prepared so as to have a final concentration of 25 uM were added and then allowed to react at 30°C for 1 hour. All wells of the plate on which reactions were completed were treated with 25 *µ*ℓ of ADP-Glo^{™} reagent and then allowed to react at 30°C for 40 minutes. After that, all wells were treated with 50 *µ*ℓ of kinase detection buffer, and then allowed to react at 30°C for 30 minutes under light shielding conditions. For the plate on which all reactions were completed, luminescence was measured and the results were calculated. Evaluation was carried out in duplicate, and negative control and positive control were calculated depending on whether or not the enzyme was added without treatment of the compound. The IC₅₀ was calculated based on the calculated values.

As a result of the calculation, the inhibitory activity against BTK (BTK IC₅₀) of the compound represented by Chemical Formula 1 prepared in Example was 0.4 nM ~1.4 nM, and the inhibitory activity against ITK (ITK IC₅₀) was 1.0 nM ~ 1.7 nM. From this, it can be confirmed that the compound represented by Chemical Formula 1 exhibits excellent BTK and ITK dual-activity inhibitory effect.

### Comparison of Examples and Reference Examples 2 to 5

In order to confirm the impurities produced together during the preparation of the compound represented by Chemical Formula 1, which is the final compound prepared in the preparation methods of the Example and Reference Examples 2 to 5, impurities were isolated using high performance liquid chromatography (HPLC) according to relative retention time (RRT) under the conditions shown in Table 1 below, and the results are shown in Table 2 below together with the yield of step 3.

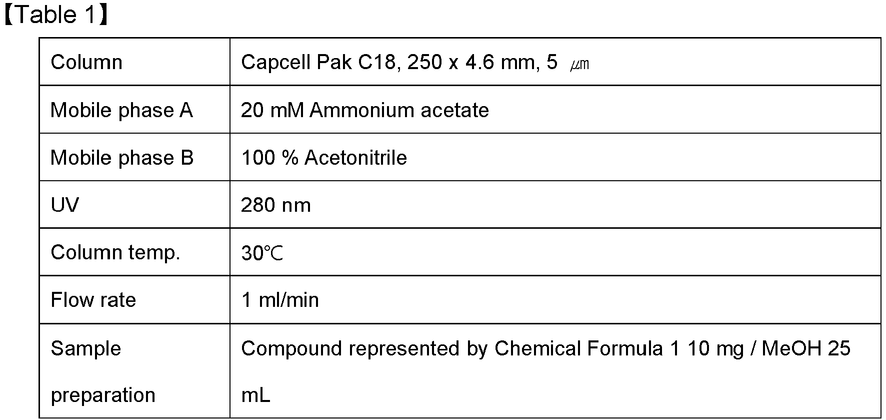

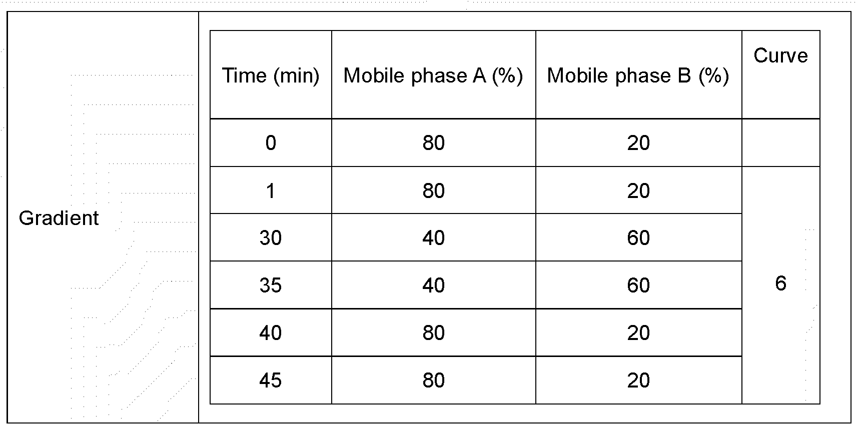

1) Impurity having a RRT of less than 1.0 (RRT<1.0)
2) Impurity A having an RRT of 1.17 (ImpA): A compound in which Compound represented by Chemical Formula 1 and Compound represented by Chemical Formula 1-4 were bonded
3) Impurity B having an RRT of 1.37 (ImpB): Dimer of the compound represented by Chemical Formula 1
4) Impurity C (ImpC) with an RRT of 1.18: Compound in which HCI was added to a double bond of the compound represented by Chemical Formula 1 and Compound in which HCI was added to a double bond of acrylamide

**[Table 2]**

| | Step 3 Yield | Purity | RRT<1.0 | ImpA | ImpB | ImpC |
|---|---|---|---|---|---|---|
| Example | 60% | 99.74% | < 0.1% | < 0.1% | < 0.1% | 0% |
| Reference Example 2 | 30% | 98.00% | 0.5% | 0.5% | - | 0.4% |
| Reference Example 3 | 35% | 98.00% | < 0.1% | 0.6% | 0.13% | 0.08% |
| Reference Example 4 | 60% | 99.00% | < 0.1% | < 0.1% | 0.30% | 0.50% |
| Reference Example 5 | 60% | 99.39% | < 0.1% | < 0.1% | 0.30% | 0% |

As shown in Table 2 above, it was confirmed that when the compound is prepared by the process of Example, a high-purity compound in which all kinds of impurities are reduced can be prepared in a high yield, as compared to the process of Reference Example 2 in which the compound is isolated and purified by primary and secondary crystallization in addition to the process of Reference Example 1 described above.

In addition, it was confirmed that when the compound is prepared by the process of Example, high-quality compounds with reduced impurities can be prepared, as compared to the processes of Reference Examples 3 to 5 in which the compound represented by Chemical Formula 1 is prepared by changing some processes in step 3 in the process of Example.

Specifically, it can be confirmed that the compound prepared according to the process of Example, in which (1) N,N-diisopropylethylamine (DIPEA) is used in the reaction of step 3, (2) the reaction temperature of step 3 is adjusted to -8 to -3°C, (3) the final compound is isolated through crystallization with ethyl acetate (EA), has the following features:
as compared to the compound prepared according to the process of Reference Example 3, in which (1) N,N-diisopropylethylamine (DIPEA) is not used in the reaction of step 3, (2) the reaction temperature of step 3 is adjusted to 0 to 5°C, and (3) the final compound is isolated through crystallization with ethyl acetate (EA) and dimethoxyethane (DME), the content of impurity A (ImpA), impurity B (ImpB) and impurity C (ImpC) is low;
as compared to the compound prepared according to the process of Reference Example 4, in which (1) N,N-diisopropylethylamine (DIPEA) is not used in the reaction of step 3, (2) the reaction temperature of step 3 is adjusted to 0 to 5°C, and (3) the final compound is isolated through crystallization with ethyl acetate (EA), the content of impurity B (ImpB) and impurity C (ImpC) is low; and
as compared to the compound prepared according to the process of Reference Example 5, in which (1) N,N-diisopropylethylamine (DIPEA) is used in the reaction of step 3, but (2) the reaction temperature of step 3 is adjusted to 0 to 5 °C, and (3) the final compound is isolated through crystallization with ethyl acetate (EA), the content of impurity B (ImpB) is low.

### Comparison of Example, Reference Example 1 and Reference Example 2

The yields of each step of the preparation methods of Example, Reference Example 1 and Reference Example 2 are shown in Table 3 below.

**[Table 3]**

| | Steps 1 & 2 | | Step 3 | | Total yield | Final purity |
|---|---|---|---|---|---|---|
| | Yield | Purity | Yield | Purity | | |
| Example | 60.3% | 99.8% | 60.0% | 99.74% | 36.0% | 99.74% |
| Reference Example 1 | 58.5% | - | 60.0% | 98.00% | 35.1% | 98.00% |
| Reference Example 2 | 58.5% | - | 30.0% | 98.00% | 17.6% | 98.00% |

As shown in Table 3 above, it was confirmed that when the compound represented by Chemical Formula 1 is prepared by the process of Example, step 1 and step 2 are performed in-situ, and isolation and purification of the compound are achieved through crystallization, which is advantageous for industrial production, unlike the process of Reference Example 1 in which a microwave device is required and isolation and purification of the prepared compound is performed through a column, and also that there is no decrease in yield compared to the process of Reference Example 1.

In addition, it can be confirmed that the process of Example can prepare the final compound in a remarkably high yield, as compared to the process of Reference Example 2 in which the isolation and purification of the compound is additionally performed by primary and secondary crystallization after the process of Reference Example 1 to increase the final purity of the compound

Therefore, it was confirmed that the compound represented by Chemical Formula 1 having high quality can be industrially mass-produced by the process of Example according to the present disclosure.

## Claims

1. A method for preparing a compound represented by the following Chemical Formula 1, comprising the steps of:
1) preparing a compound represented by the following Chemical Formula 1-3 by reacting a compound represented by the following Chemical Formula 1-1 with a compound represented by the following Chemical Formula 1-2 in the presence of a palladium catalyst and a base;
2) preparing a compound represented by the following Chemical Formula 1-4 by reacting a compound represented by the following Chemical Formula 1-3 in the presence of an acid; and
3) preparing a compound represented by the following Chemical Formula 1 by reacting a compound represented by the following Chemical Formula 1-4 with a compound represented by the following Chemical Formula 1-5 in the presence of a base, wherein the reaction is carried out at a temperature of -10°C or more and less than 0°C:

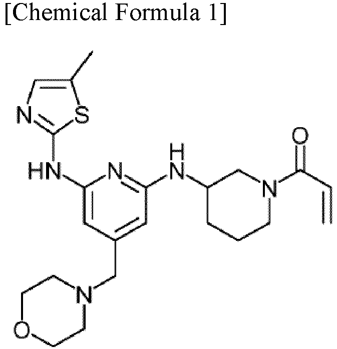

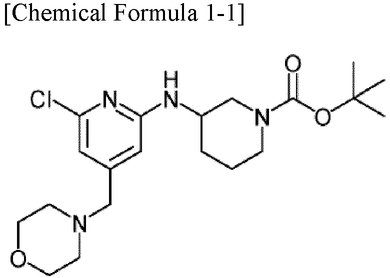

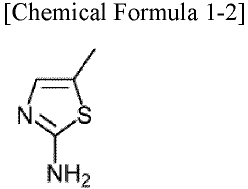

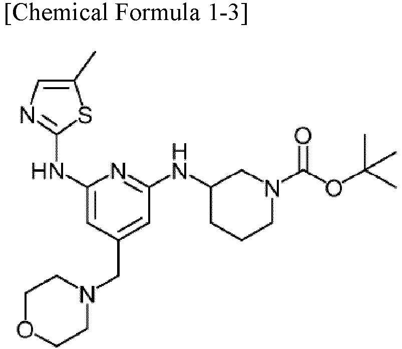

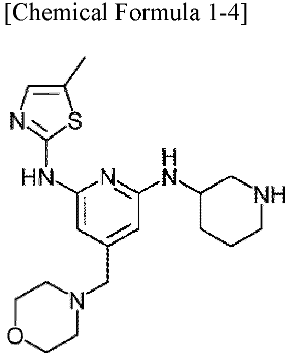

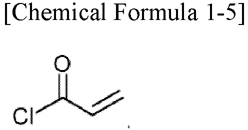

2. The method of claim 1, wherein:
in the step 1), the compound represented by Chemical Formula 1-1 and the compound represented by Chemical Formula 1-2 are used in a molar ratio of 1:0.1 to 1:2.

3. The method of claim 1, wherein:
in the step 1), the palladium catalyst is at least one selected from the group consisting of tris(dibenzylideneacetone)dipalladium(0), tetrakis(triphenylphosphine)palladium(0), bis[tris(2-methylphenyl)phosphine]palladium and palladium(II) acetate.

4. The method of claim 1, wherein:
in the step 1), the base is at least one selected from the group consisting of cesium carbonate, potassium carbonate, sodium carbonate, sodium tert-butoxide and potassium tert-butoxide.

5. The method of claim 1, wherein:
the reaction of the step 1) is carried out at a temperature of 80°C to 150°C for 3 hours to 15 hours.

6. The method of claim 1, wherein:
in the step 2), a step of crystallizing the reaction product is further included.

7. The method of claim 6, wherein:
the crystallizing step is carried out by primary crystallization with water and secondary crystallization with methanol.

8. The method of claim 1, wherein:
in the step 3), the compound represented by Chemical Formula 1-4 and the compound represented by Chemical Formula 1-5 are used in a molar ratio of 1:0.5 to 1:2.0.

9. The method of claim 1, wherein:
in the step 3), the compound represented by Chemical Formula 1-5 is added in a state of being mixed with N,N-diisopropylethylamine or triethylamine.

10. The method of claim 1, wherein:
in the step 3), a step of extracting the reaction product using ethyl acetate is further included.

11. The method of claim 10, wherein:
the extracting step is carried out at pH 6.5 to 7.5.

12. The method of claim 1, wherein:
the step 3) further comprises a step of dissolving the reaction product in tetrahydrofuran; mixing a phosphoric acid-containing solution with the prepared solution; and filtering the prepared mixture and then concentrating the filtrate under reduced pressure.

13. The method of claim 12, wherein:
the phosphoric acid-containing solution is a solution in which 0.05 to 0.5 moles of phosphoric acid relative to 1 mole of the compound represented by Chemical Formula 1-4 is dissolved in tetrahydrofuran.

14. The method of claim 1, wherein:
the step 3) further comprises a step of crystallizing the reaction product with ethyl acetate.

## Patentansprüche

1. Verfahren zur Herstellung einer Verbindung, die durch die folgende chemische Formel 1 dargestellt ist, umfassend die Schritte:
1) Vorbereiten einer Verbindung, die durch die folgende chemische Formel 1-3 dargestellt ist, durch Reaktion einer Verbindung, die durch die folgende chemische Formel 1-1 dargestellt ist, mit einer Verbindung, die durch die folgende chemische Formel 1-2 dargestellt ist, in Gegenwart eines Palladiumkatalysators und einer Base;
2) Vorbereiten einer Verbindung, die durch die folgende chemische Formel 1-4 dargestellt ist, durch Reaktion einer Verbindung, die durch die folgende chemische Formel 1-3 dargestellt ist, in der Gegenwart einer Säure; und
3) Vorbereiten einer Verbindung, die durch die folgende chemische Formel 1 dargestellt ist, durch Reaktion einer Verbindung, die durch die folgende chemische Formel 1-4 dargestellt ist, mit einer Verbindung, die durch die folgende chemische Formel 1-5 dargestellt ist, in Gegenwart einer Base, wobei die Reaktion bei einer Temperatur von -10 °C oder mehr und weniger als 0 °C durchgeführt wird:

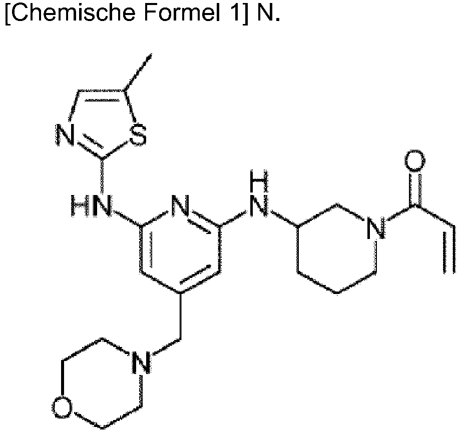

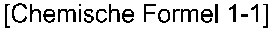

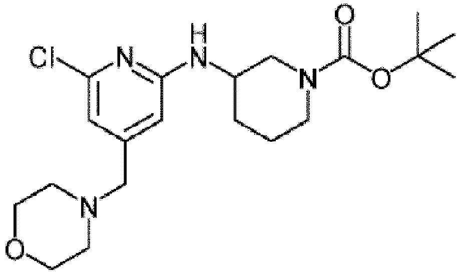

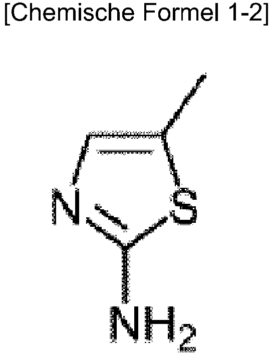

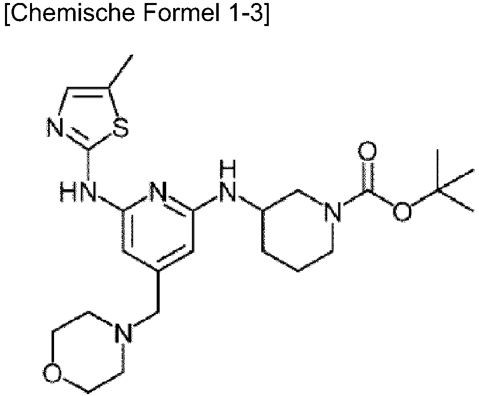

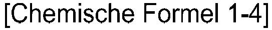

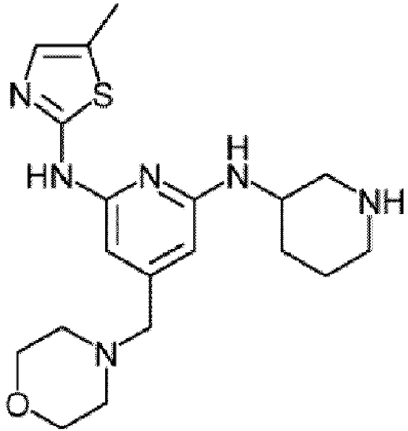

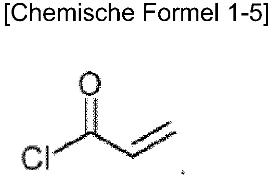

2. Verfahren nach Anspruch 1, wobei:
in Schritt 1) die Verbindung, die durch die chemische Formel 1-1 dargestellt ist, und die Verbindung, die durch die chemische Formel 1-2 dargestellt ist, in einem Molverhältnis von 1:0,1 bis 1:2 verwendet werden.

3. Verfahren nach Anspruch 1, wobei:
in Schritt 1) der Palladiumkatalysator mindestens einer ist, der aus der Gruppe ausgewählt ist, bestehend aus Tris(dibenzylideneaceton)dipalladium(0), Tetrakis(triphenylphosphin)palladium(0), Bis[tris(2-methylphenyl)phosphin]palladium und Palladium(II)-acetat.

4. Verfahren nach Anspruch 1, wobei:
in Schritt 1) die Base mindestens eine ist, die aus der Gruppe ausgewählt ist, bestehend aus Cesiumcarbonat, Kaliumcarbonat, Natriumcarbonat, Natriumtert-butoxid und Kalium-tert-butoxid.

5. Verfahren nach Anspruch 1, wobei:
die Reaktion des Schritts 1) bei einer Temperatur von 80 °C bis 150 °C für 3 Stunden bis 15 Stunden durchgeführt wird.

6. Verfahren nach Anspruch 1, wobei:
in Schritt 2) ein Schritt des Kristallisierens des Reaktionsprodukts weiter enthalten ist.

7. Verfahren nach Anspruch 6, wobei:
der Kristallisationsschritt durch primäre Kristallisation mit Wasser und sekundäre Kristallisation mit Methanol durchgeführt wird.

8. Verfahren nach Anspruch 1, wobei:
in Schritt 3) die Verbindung, die durch die chemische Formel 1-4 dargestellt ist, und die Verbindung, die durch die chemische Formel 1-5 dargestellt ist, in einem Molverhältnis von 1:0,5 bis 1:2,0 verwendet werden.

9. Verfahren nach Anspruch 1, wobei:
in Schritt 3) die Verbindung, die durch die chemische Formel 1-5 dargestellt ist, in einem Zustand hinzugefügt wird, in dem sie mit N,N-Diisopropylethylamin oder Triethylamin gemischt ist.

10. Verfahren nach Anspruch 1, wobei:
in Schritt 3) ein Schritt des Extrahierens des Reaktionsprodukts unter Verwendung von Ethylacetat weiter enthalten ist.

11. Verfahren nach Anspruch 10, wobei:
der Extraktionsschritt bei einem pH-Wert von 6,5 bis 7,5 durchgeführt wird.

12. Verfahren nach Anspruch 1, wobei:
Schritt 3) ferner einen Schritt des Auflösens des Reaktionsprodukts in Tetrahydrofuran; des Mischens einer phosphorsäurehaltigen Lösung mit der vorbereiteten Lösung; und des Filterns der vorbereiteten Mischung und des anschließenden Konzentrierens des Filtrats unter reduziertem Druck umfasst.

13. Verfahren nach Anspruch 12, wobei:
die phosphorsäurehaltige Lösung eine Lösung ist, in der 0,0,5 bis 0,5 Mol Phosphorsäure im Verhältnis zu 1 Mol der durch die chemische Formel 1-4 dargestellten Verbindung in Tetrahydrofuran gelöst ist.

14. Verfahren nach Anspruch 1, wobei:
Schritt 3) ferner einen Schritt des Kristallisierens des Reaktionsprodukts mit Ethylacetat umfasst.

## Revendications

1. Procédé de préparation d'un composé représenté par la Formule Chimique 1 suivante, comprenant les étapes de :
1) préparation d'un composé représenté par la Formule Chimique 1-3 suivante en faisant réagir un composé représenté par la Formule Chimique 1-1 suivante avec un composé représenté par la Formule Chimique 1-2 suivante en présence d'un catalyseur au palladium et d'une base ;
2) préparation d'un composé représenté par la Formule Chimique 1-4 suivante en faisant réagir un composé représenté par la Formule Chimique 1-3 suivante en présence d'un acide ; et
3) préparation d'un composé représenté par la Formule Chimique 1 suivante en faisant réagir un composé représenté par la Formule Chimique 1-4 suivante avec un composé représenté par la Formule Chimique 1-5 suivante en présence d'une base, dans lequel la réaction est réalisée à une température supérieure ou égale à -10°C et inférieure à 0°C :

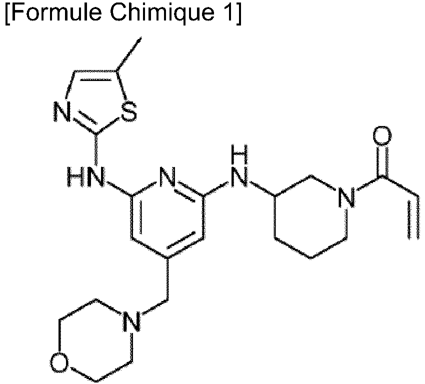

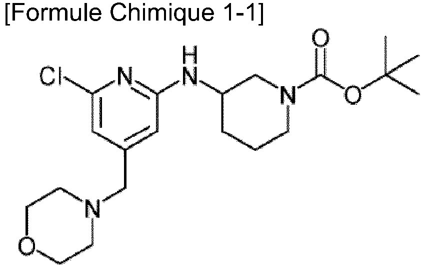

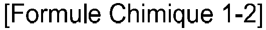

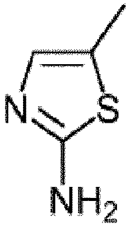

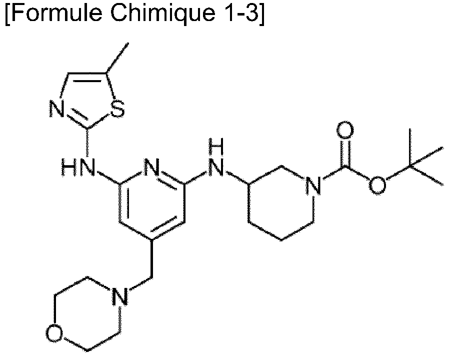

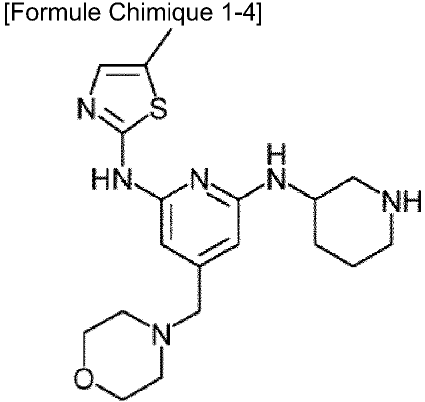

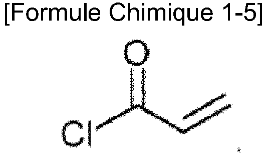

2. Procédé selon la revendication 1, dans lequel :
à l'étape 1), le composé représenté par la Formule Chimique 1-1 et le composé représenté par la Formule Chimique 1-2 sont utilisés dans un rapport molaire de 1:0,1 à 1:2.

3. Procédé selon la revendication 1, dans lequel :
à l'étape 1), le catalyseur au palladium est au moins un choisi dans le groupe constitué du tris(dibenzylidèneacétone)dipalladium(0), du tétrakis(triphénylphosphine)palladium(0), du bis[tris(2-méthylphényl)phosphine]palladium et de l'acétate de palladium(II).

4. Procédé selon la revendication 1, dans lequel :
à l'étape 1), la base est au moins un choisi dans le groupe constitué du carbonate de césium, du carbonate de potassium, du carbonate de sodium, du tert-butoxyde de sodium et du tert-butoxyde de potassium.

5. Procédé selon la revendication 1, dans lequel :
la réaction de l'étape 1) est réalisée à une température allant de 80°C à 150°C pendant une durée de 3 heures à 15 heures.

6. Procédé selon la revendication 1, dans lequel :
à l'étape 2) une étape de cristallisation du produit de réaction est en outre incluse.

7. Procédé selon la revendication 6, dans lequel :
l'étape de cristallisation est réalisée par une cristallisation primaire avec de l'eau et une cristallisation secondaire avec du méthanol.

8. Procédé selon la revendication 1, dans lequel :
à l'étape 3), le composé représenté par la Formule Chimique 1-4 et le composé représenté par la Formule Chimique 1-5 sont utilisés dans un rapport molaire de 1:0,5 à 1:2,0.

9. Procédé selon la revendication 1, dans lequel :
à l'étape 3), le composé représenté par la Formule Chimique 1-5 est ajouté à l'état mélangé avec de la N,N-diisopropyléthylamine ou de la triéthylamine.

10. Procédé selon la revendication 1, dans lequel :
à l'étape 3), une étape d'extraction du produit de réaction en utilisant de l'acétate d'éthyle est en outre incluse.

11. Procédé selon la revendication 10, dans lequel :
l'étape d'extraction est réalisée à un pH de 6,5 à 7,5.

12. Procédé selon la revendication 1, dans lequel :
l'étape 3) comprend en outre une étape consistant à dissoudre le produit de réaction dans du tétrahydrofurane ; à mélanger une solution contenant de l'acide phosphorique avec la solution préparée ; et à filtrer le mélange préparé, puis à concentrer le filtrat sous pression réduite.

13. Procédé selon la revendication 12, dans lequel :
la solution contenant de l'acide phosphorique est une solution dans laquelle 0,05 à 0,5 mole d'acide phosphorique, par rapport à 1 mole du composé représenté par la Formule Chimique 1-4, est dissoute dans du tétrahydrofurane.

14. Procédé selon la revendication 1, dans lequel :
l'étape 3) comprend en outre une étape de cristallisation du produit de réaction avec de l'acétate d'éthyle.
